# EUROPÄISCHE PATENTSCHRIFT

(11) **EP 0 960 626 B1**
(45) Veröffentlichungstag und Bekanntmachung des Hinweises auf die Patenterteilung: **15.10.2003**
(21) Anmeldenummer: 99110263.3
(22) Anmeldetag: 27.05.1999
(51) Int. Cl.: A61M 5/142, A61M 5/145

(54) **Implantierbare Vorrichtung zur Applikation einer Wirklösung und Betätigungsvorrichtung für eine Spritze zum Befüllen der Vorrichtung**
Implantable solution application device and actuator for a syringe for filling the device
Dispositif implantable pour l'application d'une solution et dispositif d'actionnement d'une seringue pour remplir le dispositif implantable

(30) Priorität: 29.05.1998 DE 19824016
(43) Veröffentlichungstag der Anmeldung: 01.12.1999
(73) Patentinhaber: Fresenius AG, 61352 Bad Homburg v.d.H. (DE)
(72) Erfinder: Steinbach, Bernd Dr., 61169 Friedberg (DE); Pieper, Walter, 61197 Florstadt (DE); Bernard, Rüdiger, 66640 Oberthal (DE)
(74) Vertreter: Luderschmidt, Schüler & Partner GbR

(56) Entgegenhaltungen:
- EP-A- 0 584 569
- EP-A- 0 612 535
- WO-A-88/10129
- WO-A-96/31246
- DE-A- 4 434 114
- US-A- 4 781 686

## Beschreibung

Die Erfindung betrifft eine Anordnung mit einer implantierbaren Vorrichtung zur Applikation einer Wirklösung und einer Betätigungsvorrichtung für eine Spritze zum Befüllen der Vorrichtung.

Zur gleichmäßigen Applikation einer Wirklösung über einen längeren Behandlungszeitraum beispielsweise im Bereich der Chemotherapie sind Infusionspumpen bekannt, die in den Körper des Patienten implantiert werden.

Die US-A-4,496,343 beschreibt eine implantierbare Infusionspumpe, die über einen Medikamentenspeicher verfügt, der in bestimmten Zeitabständen wieder befüllt werden muß. Die bekannte Infusionspumpe weist einen Gehäusekörper auf, der von einer Membran in zwei Kammern getrennt ist. Die erste Kammer enthält die zu applizierende Wirklösung, während die zweite Kammer eine isobar expandierende Treibsubstanz enthält. Zum transcutanen Nachfüllen der ersten Kammer mittels einer Injektionsspritze ist eine Gehäuseöffnung vorgesehen, die von einem durchstechbaren Septum verschlossen ist.

Um einen direkten Zugang zu dem Katheter zu schaffen, weist die bekannte Infusionspumpe eine dritte Kammer auf, die über einen Kanal mit der ersten Kammer zur Aufnahme der Wirklösung verbunden ist. Die Wirklösung strömt aus der ersten Kammer über den Kanal in die dritte Kammer und aus der dritten Kammer über einen Katheter zu der Infusionsstelle. Die dritte Kammer erlaubt eine zusätzliche Menge an Wirklösung direkt zu applizieren oder eine Probe zu entnehmen. Zur Applikation der Wirklösung oder Probenentnahme mittels einer Injektionsspritze ist eine zweite Gehäuseöffnung vorgesehen, die von einem zweiten durchstechbaren Septum verschlossen ist.

Lebensbedrohliche Situationen können für den Patienten bei der Verwechslung der beiden Septen entstehen. Dies ist dann gegeben, wenn die für die erste Kammer vorgesehene Menge an Wirklösung ganz oder teilweise in die dritte Kammer für die Bolusinfusion oder Probenentnahme gespritzt wird. Dies kann beispielsweise bei Morphium zum sofortigen Atemstillstand führen. Die Verwechslung der beiden Septen ist in der Praxis deshalb relativ leicht möglich, weil diese sich unter der Haut befinden.

Die EP-A-612 535 beschreibt eine implantierbare Vorrichtung mit zwei Kammern unterschiedlicher Größe, die sich separat befüllen lassen. Die große Kammer dient zur Aufnahme einer ersten und die kleine Kammer zur Aufnahme einer zweiten Infusionslösung, die miteinander vermischt werden sollen. Beide Kammern werden über eine gemeinsame Öffnung befüllt. Zum Befüllen der kleinen Kammer ist eine Spritze mit einer Nadel vorgesehen, die eine seitliche Öffnung aufweist. Diese Öffnung liegt genau auf der Höhe der Füllöffnung des zu der kleinen Kammer führenden Füllkanals. Um zu verhindern, daß im Falle eines Defekts Infusionslösung aus der großen Kammer über den Füllkanal in die kleine Kammer gelangt, ist in dem Füllkanal ein Rückschlagventil angeordnet. Bei der bekannten Infusionsvorrichtung werden beide Kammern nicht über getrennte Septen befüllt.

WO-A-88 10129 zeigt einen Halter für eine Spritze der die Dauer einer Injektion verlängern kann.

Eine Betätigungsvorrichtung für eine Spritze ist aus der WO 88/10129 bekannt. Die Betätigungsvorrichtung verfügt über eine Halterung für die Spritze und ein Betätigungsorgan, mit dem der Kolben der Spritze mit einer vorgegebenen Kraft beaufschlagt wird.

Der Erfindung liegt die Aufgabe zugrunde, die Sicherheit von implantierbaren Infusionspump weiter zu erhöhen, die über ein zusätzliches Septum für eine Bolusinfusion verfügen.

Die Lösung dieser Aufgabe erfolgt erfindungsgemäß mit den im Patentanspruch 1 angegebene Merkmalen.

Die Vorrichtung zur Applikation einer Wirklösung der erfindungsgemäßen Anordnung weist eine Einrichtung auf, die den Strömungsweg von der Kammer für die Bolusinfusion und der Stelle, an der die Wirklösung zu applizieren ist, nur dann freigibt, wenn der Wirklösungsbolus der Kammer unter einem Druck zugeführt wird, der über einem vorgegebenen Minimaldruck liegt.

Der Eintritt lebensbedrohlicher Situationen durch die plötzliche Applikation einer größeren Menge an Wirklösung ist dann ausgeschlossen, wenn zum Nachfüllen des Medikamentenspeichers die Wirklösung immer nur unter einem Druck zugeführt wird, der unter dem Minimaldruck liegt. Wenn das Septum des Medikamentenspeichers mit dem Septun für die Bolusinfusion verwechselt werden sollte, kann die Wirklösung nicht appliziert werden, da die Fluidverbindung zwischen der Kammer für die Bolusinfusion und der Infusionsstelle nicht freigegeben ist.

Die den Strömungsweg zwischen der Kammer für die Bolusinfusion und der Infusionsstelle erst bei Überschreiten eines vorgegebenen Minimaldrucks freigebende Einrichtung verhindert nicht nur unbeabsichtigte Infusionen sondern auch, daß Wirklösung in die Kammer für die Bolusinfusion gelangt.

In einer bevorzugten Ausführungsform ist die Einrichtung zur Freigabe des Strömungsweges ein Überdruckventil, das vorteilhafterweise als geschlitzte Scheibe, vorzugsweise aus Silikon, mit einem Öffnungsdruck von 2 bis 4 bar ausgebildet ist.

Die Betätigungsvorrichtung der erfindungsgemäßen Anordnung verhindert, daß beim Befüllen des Medikamentenspeichers die Wirklösung mit einem Druck zugeführt wird, der über dem vorgegebenen Minimaldruck liegt. Zum Wiederauffüllen des Medikamentenspeichers wird in die Betätigungsvorrichtung eine herkömmliche Spritze eingelegt. Die Betätigungsvorrichtung weist hierzu eine Halterung zur Befestigung des Zylinders der Spritze und ein Betätigungsorgan zum Erfassen des Kolbens der Spritze sowie eine Antriebseinrichtung zum Verschieben des Betätigungsorgans auf, so daß der Kolben in den Zylinder der Spritze gedrückt wird.

Zur Aktivierung der Betätigungsvorrichtung wird das Betätigungsorgan entgegen einer definierten Rückstellkraft vorgespannt, durch die das Betätigungsorgan zum Entleeren der Spritze zurückgeschoben wird. Die Rückstellkraft ist derart bemessen, daß die Wirklösung aus der Spritze mit einem Druck zugeführt wird, der unter dem Minimaldruck der Einrichtung zur Freigabe des Strömungsweges zwischen der Kammer für die Bolusinfusion und der Infusionsstelle liegt. Der Druck, unter dem die Wirklösung zugeführt wird, liegt aber über dem Treibmitteldruck im Medikamentenspeicher der Vorrichtung zur Applikation der Wirklösung. Ein typischer Arbeitsbereich liegt zwischen 1 und 1,5 bar.

Wenn sich die Spitze der in die Betätigungsvorrichtung eingelegten Spritze beim Wiederauffüllen des Medikamentenspeichers unter dem Septum der Kammer zur Aufnahme der Wirklösung befindet, wird der Medikamentenspeicher gefüllt. Befindet sich die Nadelspitze hingegen unter dem Septum der Kammer zur Aufnahme des Wirklösungsbolus, wird eine Fehlinfusion wirksam verhindert. Ebenfalls wird eine Infusion verhindert, wenn sich die Nadelspitze in einem der Septen befindet.

Zur Verabreichung eines Wirklösungsbolus wird die gebräuchliche Spritze zur Aufnahme der Wirklösung von Hand betätigt, um einen Druck aufzubauen, der über dem vorgegebenen Minimaldruck liegt.

Im folgenden werden ein Ausführungsbeispiel der Vorrichtung zur Applikation einer Wirklösung sowie der Betätigungsvorrichtung für eine Spritze zum Befüllen der Vorrichtung unter Bezugnahme auf die Zeichnungen näher erläutert.

Es zeigen:

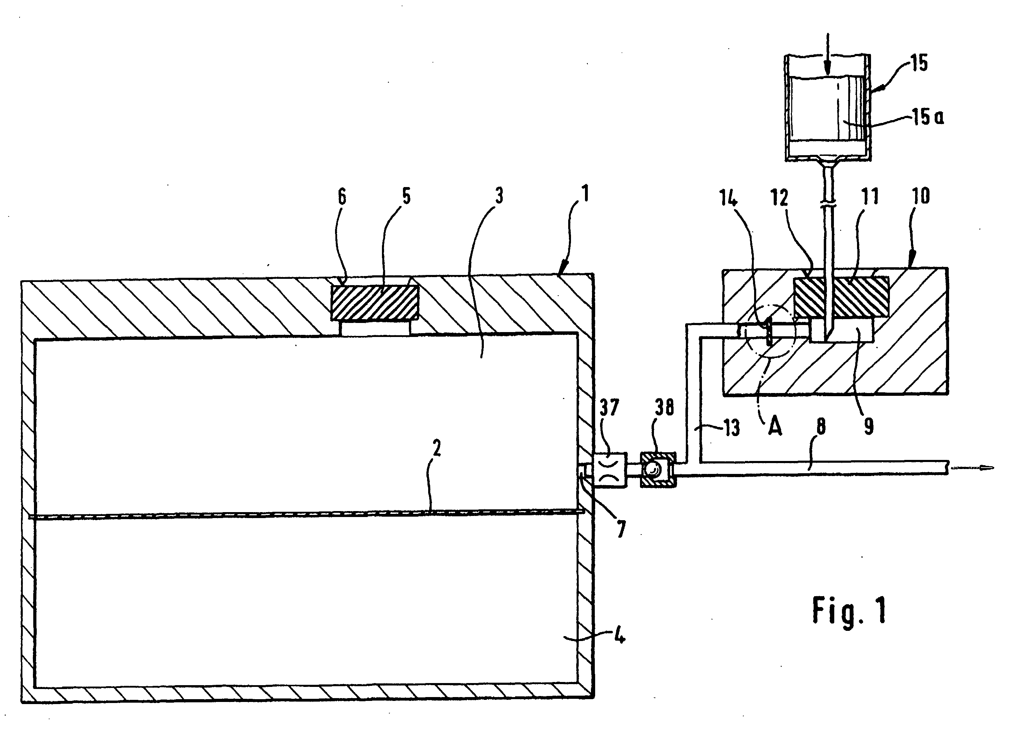

- Figur 1: ein Ausführungsbeispiel der implantierbaren Vorrichtung zur Applikation einer Wirklösung der erfindungsgemäßen Anordnung in schematischer Darstellung,
- Figur 2: den Ausschnitt A von Figur 1 in vergrößerter Darstellung und
- Figur 3: eine Prinzipskizze eines Ausführungsbeispiels der Betätigungsvorrichtung der erfindungsgemäßen Anordnung für eine Spritze zum Befüllen der Vorrichtung zur Applikation einer Wirklösung.

Die Vorrichtung zur Applikation einer Wirklösung, insbesondere eines Medikaments, weist einen implantierbaren Gehäusekörper 1 auf, der durch eine flexible Membran 2 in eine erste Kammer 3 und eine zweite Kammer 4 geteilt ist.

Die erste Kammer 3 nimmt das zu applizierende Medikament auf während die zweite Kammer 4 eine isobar expandierende Treibsubstanz enthält.

Die erste Kammer weist eine mit einem durchstechbaren Septum 5 verschlossene Zuführöffnung 6 auf, um die Kammer mittels einer Injektionsspritze mit dem Medikament transcutan befüllen zu können. Ferner weist die erste Kammer 3 einen Auslaß 7 auf, an den eine zu der Infusionsstelle führende Infusionsleitung 8 angeschlossen ist. Da die isobar expandierende Treibsubstanz in der zweiten Kammer einen gleichmäßigen Druck auf die flexible Membran 2 ausübt, strömt das Medikament aus der ersten Kammer 3 durch die Infusionsleitung 8 zu der Infusionsstelle. Zur Einstellung der Strömungsrate ist in der Infusionsleitung 8 eine Drossel 37 angeordnet.

Zur Verabreichung eines Medikamentenbolus ist eine dritte Kammer 9 in einem zweiten Gehäusekörper 10, der ebenfalls unter der Haut des Patienten angeordnet wird, vorgesehen. Die dritte Kammer 9 weist eine mit einem zweiten durchstechbaren Septum 11 verschlossene Zuführöffnung 12 auf. Stromab des Auslasses 7 zweigt von der Infusionsleitung 8 eine Zuführleitung 13 ab, die an der dritten Kammer 9 angeschlossen ist. In der Zuführleitung 13 ist ein Überdruckventil 14 angeordnet, das den Strömungsweg von der dritten Kammer 9 zu der Infusionsstelle dann freigibt, wenn der Medikamentenbolus unter einem Druck zugeführt wird, der oberhalb eines vorgegebenen Minimaldrucks liegt. Der Druck bei dem das Ventil 14 öffnet, liegt vorzugsweise zwischen 2,5 und 3,5 bar.

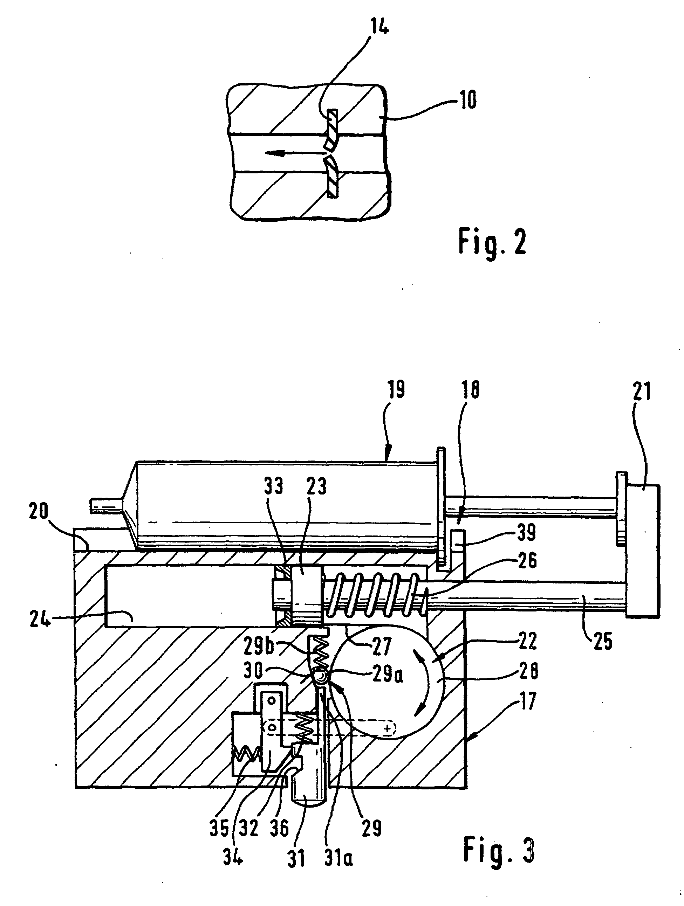

Figur 2 zeigt das Überdruckventil 14 in vergrößerter Darstellung. Das Überdruckventil ist als geschlitzte Silikonscheibe ausgebildet, die in den zweiten Gehäusekörper 10 eingesetzt ist.

Zur Verabreichung eines Medikamentenbolus wird die dritte Kammer 9 mittels einer herkömmlichen Injektionsspritze 15, deren Nadel durch das zweite Septum 11 gestochen wird, mit dem Medikament befüllt. Durch Betätigung des Kolbens 15a der Injektionsspritze 15 wird ein Druck aufgebaut, der über dem vorgegebenen Minimaldruck liegt, bei dem das Überdruckventil 14 öffnet. Der Medikamentenbolus strömt aus der dritten Kammer 9 durch die Zuführleitung 13 und die Infusionsleitung 8 zu der Infusionsstelle. Ein Rückfluß des Medikamentenbolus in die erste Kammer 3 wird durch ein Rückschlagventil 38 verhindert, das in der Infusionsleitung 8 zwischen dem Auslaß 7 der ersten Kammer und der Zuführleitung 13 angeordnet ist.

Figur 3 zeigt eine Betätigungsvorrichtung für eine herkömmliche Spritze zum Nachfüllen der ersten Kammer 3 der Vorrichtung zur Applikation eines Medikaments.

Die Betätigungsvorrichtung weist einen Gehäusekörper 17 mit einer Halterung 18 auf, an der eine herkömmliche Injektionsspritze 19 befestigt wird. Die Halterung 18 umfaßt eine muldenförmige Vertiefung 20 für den Zylinder der Spritze 19 und einen vorspringenden Ansatz 39, an dem die Spritze an dem oberen Rand des Zylinders fixiert wird.

Darüber hinaus verfügt die Betätigungsvorrichtung über ein an dem Kolben der Spritze angreifendes Betätigungsorgan 21, das von einer Antriebseinrichtung 22 verschoben wird, so daß der Kolben in den Zylinder der Spritze gedrückt wird.

Die Antriebseinrichtung 22 für das Betätigungsorgan 21 umfaßt einen Kolben 23, der in einem Zylinderraum 24 in dem Gehäusekörper 17 verschiebbar gelagert ist. An dem Kolben 23 ist eine sich aus dem Gehäusekörper erstreckende Kolbenstange 25 befestigt, an der wiederum das Betätigungsorgan 21 für den Kolben der Spritze 19 befestigt ist.

Vor dem Einlegen der Spritze 19 wird der Kolben entgegen der Rückstellkraft einer in dem Zylinderraum 24 angeordneten Druckfeder 26 mit einem an dem Kolben 23 befestigten Zugseil 27 vorgespannt, das auf einer Seilscheibe 28 aufgewickelt ist, die in dem Gehäusekörper 17 angeordnet ist. In der vorgespannten Stellung wird die Seilscheibe 28 von einer Rücklaufsperre 29 arretiert. Die Rücklaufsperre 29 weist eine Kugel 29a auf, die von einer Druckfeder 29b in einen Spalt zwischen der Seilscheibe 28 und einer schräg verlaufenden Anlagefläche 30 gedrückt wird, so daß die Seilscheibe klemmend fixiert ist. Die Rücklaufsperre 29 wird mit einem Druckknopf 31 gelöst, der mit einer Druckfeder 32 federnd vorgespannt ist und sich mit einem vorspringenden Ansatz 31a an der Kugel 29a der Rücklaufsperre 29 abstützt. Wenn der Druckknopf 31 entgegen der Kraft der Feder 32 in den Gehäusekörper 17 gedrückt wird, drückt der vorspringende Ansatz 31a die Kugel 29a der Rücklaufsperre 29 zurück, so daß die Seitscheibe 28 frei ist und das Betätigungsorgan 21 durch die Rückstellkraft der Druckfeder 26 in dem Zylinderraum 24 zum Entleeren der Spritze 19 vorgeschoben wird. In der die Seilscheibe freigebenden Stellung wird der Druckknopf 31 von einer Halteklinke 34 gehalten, die mit einer Druckfeder 35 federnd vorgespannt ist und in eine Ausnehmung 36 des Druckknopfs 31 einklinkt.

Damit das Betätigungsorgan 21 nach Lösen der Rücklaufsperre 29 nicht plötzlich zurückschnellt, ist an der Stirnseite des Kolbens 23 ein sich an der Wandung des Zylinderraums 24 abstützendes Dämpfungselement 33 vorgesehen.

Die Rückstellkraft der in dem Zylinderraum 24 angeordneten Druckfeder 26 ist derart bemessen, daß der Arbeitsdruck der Spritze 19 zwischen 1 und 1,5 bar liegt. Da dieser Druck unter dem Druck liegt, bei dem die Strömungsverbindung zwischen der dritten Kammer zur Aufnahme des Medikamentenbolus und der Infusionsstelle liegt, ist ausgeschlossen, daß eine zum Wiederauffüllen der Medikamentenkammer bestimmte Wirklösung direkt zu dem Patienten gelangt.

## Patentansprüche

1. Anordnung mit einer implantierbaren Vorrichtung (1) zur Applikation einer Wirklösung und einer Betätigungsvorrichtung (17) für eine Spritze zum Befüllen der implantierbaren Vorrichtung,
wobei die implantierbare Vorrichtung aufweist:
eine Kammer (3) zur Aufnahme der zu applizierenden Wirklösung, die eine mit einem durchstechbaren Septum (5) verschlossene Nachfüllöffnung (6) aufweist.
eine Kammer (9) zur Aufnahme eines Wirklösungsbolus, die eine mit einem durchstechbaren Septum (11) verschlossene Zuführöffnung (12) aufweist,
Mittel (8) zur Herstellung einer Fluidverbindung zwischen der Kammer (3) zur Aufnahme der zu applizierenden Wirklösung und einer Infusionsstelle, an der die Wirklösung zu applizieren ist,
Mittel (13) zur Herstellung einer Fluidverbindung zwischen der Kammer (9) zur Aufnahme des Wirklösungsbolus und der Infusionsstelle und
Mittet (4) zum Fördern der Wirklösung aus der Kammer (3) zur Aufnahme der zu applizierenden Wirklösung zu der Infusionsstelle,
wobei die Mittel (13) zur Herstellung einer Fluidverbindung zwischen der Kammer (9) zur Aufnahme des Wirklösungsbolus und der Infusionsstelle eine Einrichtung (14) umfassen, die den Strömungsweg dann freigibt, wenn der Wirklösungsbolus der Kammer (9) unter einem Druck zugeführt wird, der über einem vorgegebenen Minimaldruck liegt,
und
wobei die Betätigungsvorrichtung aufweist:
eine Halterung (18) zur Befestigung des Zylinders der Spritze und ein Betätigungsorgan (21) zum Erfassen des Kolbens der Spritze,
eine Antriebseinrichtung (22) zum Verschieben des Betätigungsorgans, die derart ausgebildet ist, daß das Betätigungsorgan (21) aus einer ersten Position entgegen einer vorgegebenen Rückstellkraft (26) in eine zweite Position verschiebbar und in dieser Position arretierbar ist, und
eine das Betätigungsorgan freigebende Auslöseeinrichtung (29), so daß das Betätigungsorgan nach der Freigabe durch die vorgegebene Rückstellkraft in die erste Position zurückgeschoben wird,
**dadurch gekennzeichnet,**
**daß** die vorgegebene Rückstellkraft, derart bemessen ist, daß die Wirklösung mit der Spritze mit einem Druck zuführbar ist, der unter dem vorgegebenen Minimaldruck der Einrichtung (14) zur Freigabe des Strömungsweges zwischen der Kammer (9) zur Aufnahme des Wirklösungsbolus und der Infusionsstelle liegt.

2. Anordnung nach Anspruch 1, **dadurch gekennzeichnet, daß** die Mittel zur Herstellung einer Fluidverbindung zwischen der Kammer (3) zur Aufnahme der zu applizierenden Wirklösung und der Infusionsstelle eine von der Kammer (3) abgehende Infusionsleitung (8) und die Mittel zur Herstellung der Fluidverbindung zwischen der Kammer (9) zur Aufnahme des Wirklösungsbolus und der Infusionsstelle eine von der Kammer (9) abgehende und in die Infusionsleitung mündende Zuführleitung (13) umfassen und **daß** die Einrichtung zur Freigabe des Strömungsweges ein in der Zuführleitung angeordnetes Überdruckventil (14) ist.

3. Anordnung nach Anspruch 2, **dadurch gekennzeichnet, daß** das Überdruckventil (14) eine geschlitzte Scheibe ist.

4. Anordnung nach Anspruch 3, **dadurch gekennzeichnet, daß** die geschlitzte Scheibe (14) aus Silikon besteht.

5. Anordnung nach einem der Ansprüche 1 bis 4, **dadurch gekennzeichnet, daß** der Minimaldruck zwischen 2 und 4 bar, vorzugsweise zwischen 2,5 und 3,5 bar, liegt.

6. Anordnung nach einem der Ansprüche 1 bis 5, **dadurch gekennzeichnet, daß** das Betätigungsorgan (21) mit einem Federelement (26) vorgespannt ist.

7. Anordnung nach einem der Ansprüche 1 bis 6, **dadurch gekennzeichnet, daß** Mittel (33) zum Dämpfen der Bewegung des Betätigungsorgans (21) vorgesehen sind.

## Claims

1. Arrangement with an implantable device (1) for the application of an active solution and an actuator (17) for a syringe for filling the implantable device. whereby the implantable device has:
a chamber (3) to take the active solution to be applied which has a filling aperture (6) sealed with a septum (5) which can be pierced.
a chamber (9) to take an active solution bolus which has a feeder aperture (12) sealed with a septum (11) which can be pierced,
means (8) to make a fluid connection between the chamber (3) to take the active solution to be applied and an infusion point at which the active solution is to be applied,
means (13) to make a fluid connection between the chamber (9) to take the active solution bolus and the infusion point and
means (4) to convey the active solution from the chamber (3) to take the active solution to be applied to the infusion point,
whereby the means (13) to make a fluid connection between the chamber (9) to take the active solution bolus and the infusion point includes a device (14) which frees the flow path when the active solution bolus is fed to the chamber (9) under a pressure which is above a specified minimum pressure,
and
whereby the actuator has:
a mounting (18) to hold the cylinder of the syringe and an actuator (21) to hold the plunger of the syringe,
a propulsion device (22) to push the actuator which is designed so that the actuator (21) can be moved from a first position against a specified reset force (26) into a second position and locked in this position, and
a release device (29) freeing the actuator so that after release the a ctuator is pushed back into the first position by the specified reset force,
**characterised in that**
the specified reset force is calculated such that -the active solution can be supplied with the syringe at a pressure which is less than the specified minimum pressure of the device (14) to free the flow path between the chamber (9) to take the active solution bolus and the infusion point.

2. Arrangement according to claim 1 **characterised in that** the means to make a fluid connection between the chamber (3) to take the active solution to be applied and the infusion point includes an infusion pipe (8) leading from the chamber (3) and the means to make the fluid connection between the chamber (9) to take the active solution bolus and the infusion point includes a feeder pipe (13) leading from the chamber (9) and opening into the infusion pipe and that the device to free the flow path is an overpressure valve (14) located in the feeder pipe.

3. Arrangement according to claim 2 **characterised in that** the overpressure valve (14) is a slotted disc.

4. Arrangement according to claim 3 **characterised in that** the slotted disc (14) is made of silicone.

5. Arrangement according to any of claims 1 to 4 **characterised in that** the minimum pressure is between 2 and 4 bar, preferably between 2.5 and 3.5 bar.

6. Arrangement according to any of claims 1 to 5 **characterised in that** the actuator (21) is pre-tensioned with a spring element (26).

7. Arrangement according to any of claims 1 to 6 **characterised in that** means (33) are provided to cushion the movement of the actuator (21).

## Revendications

1. Agencement comprenant un dispositif (1) implantable, destiné à l'administration d'une solution active et un dispositif de commande (17) pour une seringue utilisée pour remplir le dispositif implantable,
dans lequel le dispositif implantable comporte :
une chambre (3) destinée à recevoir la solution active à administrer, qui comporte un orifice de remplissage (6) obturé par un septum (5) à transpercer,
une chambre (9) destinée à recevoir un bol de solution active, qui comporte un orifice de remplissage (12) obturé par un septum (11) à transpercer,
des moyens (8) destinés à réaliser une liaison fluidique entre la chambre (3), destinée à recevoir la solution active à administrer, et une zone d'infusion, au niveau de laquelle doit être administrée la solution active,
des moyens (13) destinés à réaliser une liaison fluidique entre la chambre (9), destinée à recevoir un bol de solution active, et la zone d'infusion et
des moyens (4) destinés à transporter la solution active hors de la chambre (3), destinée à recevoir la solution active à administrer, vers la zone d'infusion,
dans lequel les moyens (13) destinés à réaliser une liaison fluidique entre la chambre (9), destinée à recevoir un bol de solution active, et la zone d'infusion comportent une unité (14), qui libère la voie d'écoulement lorsque le bol de solution active est acheminé dans la chambre (9) sous une pression supérieure à une pression minimum prédéfinie,
dans lequel le dispositif de commande comporte :
un support (18) pour fixer le cylindre de la seringue et un organe de commande (21) destiné à manoeuvrer le piston de la seringue,
une unité d'entraînement (22) pour déplacer l'organe de commande, laquelle est conçue de telle sorte que l'organe de commande (21) peut être déplacé à partir d'une première position à l'encontre d'une force de rappel (26) prédéfinie dans une deuxième position et peut être bloqué dans cette position, et
une unité de dégagement (29) libérant l'organe de commande, de telle sorte que l'organe de commande après libération est ramené par la force de rappel prédéfinie dans la première position,
**caractérisé en ce que**
la force de rappel prédéfinie est définie de telle sorte que la solution active peut être acheminée avec la seringue sous une pression qui est inférieure à la pression minimum prédéfinie de l'unité (14) destinée à libérer la voie d'écoulement entre la chambre (9), destinée à recevoir un bol de solution active, et la zone d'infusion.

2. Agencement selon la revendication 1, **caractérisé en ce que** les moyens destinés à réaliser une liaison fluidique entre la chambre (3), destinée à recevoir la solution active à administrer, et la zone d'infusion comportent une conduite d'infusion (8) partant de la chambre (3), et les moyens destinés à réaliser une liaison fluidique entre la chambre (9), destinée à recevoir un bol de solution active, et la zone d'infusion comportent une conduite d'admission (13) partant de la chambre (9) et débouchant dans la conduite d'infusion, et **en ce que** l'unité destinée à libérer la voie d'écoulement est une soupape à surpression (14) agencée dans la conduite d'admission.

3. Agencement selon la revendication 2, **caractérisé en ce que** la soupape à surpression (14) est un disque fendu.

4. Agencement selon la revendication 3, **caractérisé en ce que** le disque fendu (14) est réalisé en silicone.

5. Agencement selon l'une quelconque des revendications 1 à 4, **caractérisé en ce que** la pression minimum se situe entre 2 et 4 bars, de préférence entre 2,5 et 3,5 bars.

6. Agencement selon l'une quelconque des revendications 1 à 5, **caractérisé en ce que** l'organe de commande (21) est précontraint par un ressort (26) .

7. Agencement selon l'une quelconque des revendications 1 à 6, **caractérisé en ce que** des moyens (33) destinés à amortir le mouvement de l'organe de commande (21) sont prévus.
